**Europäisches Patentamt**

**European Patent Office**

**Office européen des brevets**

(19)

(11) Veröffentlichungsnummer : **0 289 556 B1**

(12) # EUROPÄISCHE PATENTSCHRIFT

(45) Veröffentlichungstag der Patentschrift :
**15.01.92 Patentblatt 92/03**

(51) Int. Cl.$^5$ : **G01N 33/545**

(21) Anmeldenummer : **87907318.7**

(22) Anmeldetag : **31.10.87**

(86) Internationale Anmeldenummer :
**PCT/EP87/00649**

(87) Internationale Veröffentlichungsnummer :
**WO 88/03648 19.05.88 Gazette 88/11**

(54) **BIOCHEMISCHES REAKTIONSGEFÄSS.**

(30) Priorität : **03.11.86 DE 3637421**

(43) Veröffentlichungstag der Anmeldung :
**09.11.88 Patentblatt 88/45**

(45) Bekanntmachung des Hinweises auf die
Patenterteilung :
**15.01.92 Patentblatt 92/03**

(84) Benannte Vertragsstaaten :
**AT BE CH DE FR GB IT LI LU NL SE**

(56) Entgegenhaltungen :
**EP-A- 97 573**
**EP-A- 126 392**
**EP-A- 164 889**
**EP-A- 175 195**
**FR-A- 2 332 287**
**CHEMICAL ABSTRACTS, vol. 79, 27 Nov 1973,**
**Columbus, OH (US); p. 71, no. 67731p**

(73) Patentinhaber : **NERBE, Jürgen F.**
**Ansgarstrasse 8**
**W-2105 Seevetal 11 (DE)**
Patentinhaber : **WÖNNE, Karl-Georg**
**Zum dicken Busch 12 b**
**W-2000 Barsbüttel (DE)**

(72) Erfinder : **NERBE, Jürgen F.**
**Ansgarstrasse 8**
**W-2105 Seevetal 11 (DE)**
Erfinder : **WÖNNE, Karl-Georg**
**Zum dicken Busch 12 b**
**W-2000 Barsbüttel (DE)**

(74) Vertreter : **Schaefer, Konrad**
**Gehölzweg 20**
**W-2000 Hamburg 70 (DE)**

**Beschreibung**

Die Erfindung betrifft ein Reaktionsgefäß aus chemisch inertem thermoplastischem Kunststoff zur Durchführung chemischer Reaktionen mit biospezifischen Verbindungen vorzugsweise für die medizinische Laboratoriumsdiagnostik.

Reaktionsgefäße der eingangs genannten Art finden im biologischen Labor bzw. bei biochemischen Produktions-verfahren und insbesondere in der klinischen Labordiagnostik Anwendung. Es werden darin biochemische Reaktionen der unterschiedlichsten Art durchgeführt, wobei chemische Reaktionen mit biospezifischen Verbindungen verwendet werden, also wenigstens einer der Reaktionspartner ein Eiweißkörper ist, also ein komplettes Eiweißmolekül oder ein Fragment davon. In großem Umfange werden derartige Analysen an Blutproben durchgeführt, beispielsweise zur Untersuchung von Patienten auf Krankheitsanzeichen oder in Blutbanken zur Analyse von Spenderblut. Die verwendeten chemischen Reaktionen sind unterschiedlichster Art, beispielsweise Enzymreaktionen, Hormonreaktionen, Antigen-Antikörperreaktionen oder die Blutgerinnungsreaktion, die meist am Anfang einer Blutuntersuchung steht.

Derartige Reaktionsgefäße werden heutzutage in großer Zahl zu geringen Preisen benötigt, um beispielsweise in klinischen Großlabors heute üblicher Größe kommerziell einsetzbar zu sein.

Als Werkstoff für die Reaktionsgefäße hat sich daher thermoplastischer Kunststoff der eingangs genannten Art durchgesetzt, aus dem Reaktionsgefäße kostengünstig herstellbar sind und der aufgrund seiner chemisch inerten Eigenschaften die im Gefäß ablaufenden Reaktionen nicht beeinflußt.

In derartigen Reaktionsgefäßen muß nach dem Stand der Technik der zur Durchführung einer biochemischen Reaktion am Probematerial erforderliche Reaktionspartner zugegeben werden. Dies erfordert in nachteiliger Weise einen zusätzlichen Arbeitsgang. Weiterhin ist sehr häufig von Nachteil, daß der zugegebene Reaktionspartner vor Durchführung nachfolgender Untersuchungen - weil dann störend - wieder abgeschieden werden muß, wofür wiederum zusätzliche Arbeits-gänge erforderlich sind.

EP-A-0 164 889 beschreibt eine Substratoberfläche, die aus einem Polymer besteht. Das Polymer enthält wenigstens ein Monomer mit reaktiven Atomgruppen. Folglich liegen diese reaktiven Atomgruppen auch an der Oberfläche frei, und es können an diesen reaktiven Atomgruppen die anzubindenden biospezifischen Verbindungen direkt oder über Spacer angebunden werden. Das in dieser Schrift beschriebene Polymer des Substrats enthält also ein Monomer mit reaktiven Atomgruppen.

FR-A-2 332 287 beschreibt auch einen Kunststoff der aus einem Polymer besteht, von dem das eine Monomer ein Vinylglykol ist und das andere Monomer aus einer Reihe von Substanzen bestehen kann. Dabei muß das dem Vinylglykol beigegebene Comonomer aktive Atomgruppen zur Verfügung stellen um biologisch aktive Substansen dort anbinden zu können.

Den genannten Dokumenten kann man also entnehmen, daß man an einem Kunststoffsubstrat mit reaktivem Comonomer biospezifische Verbindungen anbinden kann.

Diese Kunststoffe sind aber sehr instabil und keineswegs chemisch inert. Es ist weiterhin nicht bekannt, solche Kunststoffe auf einem Extruder zu verarbeiten und daraus Reaktionsgefäße herzustellen.

Die Aufgabe der vorliegenden Erfindung besteht daher darin, ein Reaktionsgefäß der eingangs genannten Art zu schaffen, mit dem biochemische Reaktionen bei geringerem Zeit- und Arbeitsaufwand durchgeführt werden können.

Diese Aufgabe wird erfindungsgemäß dadurch gelöst daß der Kunststoff durch vor seiner thermoplastischen Ausformung erfolgte Beigabe einer nicht lässig inkorporierbaren Substanz derart ausgebildet ist, daß nach dem Ausformen des Kunststoffes wenigstens an der Gefäßinnenfläche inkorporierte Substanzmoleküle mit reaktiten Atomgruppen freiliegen. Erfindungsgemäß ist dem Kunststoff des Reaktionsgefäßes eine Substanz beigegeben, die im Kunststoff bei dessen Aufschmelzen vor der thermoplastischen Ausformung nicht lässig inkorporiert wird. Unter "nicht lässig" versteht man in der vorliegenden Technologie, daß die zugegebene Substanz derart an den Kunststoff gebunden ist, daß sie nicht, auch nicht in kleinen Mengen, an die Probe abgegeben wird. Die beigegebene Substanz weist Moleküle mit chemisch reaktiven Atomgruppen auf. Die Inkorporierung in den Kunststoff erfolgt derart, daß wenigstens eine größere Zahl der reaktiven Atomgruppen der zugegebenen Substanzmoleküle in der Oberfläche des Kunststoffes freiliegen. Das erfindungsgemäße Reaktionsgefäß weist daher auf seiner Innenseite in Kontakt mit der eingegebenen Probe reaktive Atomgruppen auf, die unmittelbar zur gewünschten Reaktion eingesetzt werden können oder die Anbindung eines gewünschten Liganden erlauben, welcher mit der Probe die gewünschte Reaktion durchführt. Der wesentliche Vorteil hieran ist, daß die die reaktiven Atomgruppen tragenden Substanzmoleküle bzw. die an diese gebundenen Liganden zwar zur Reaktion zur Verfügung stehen, aber nicht in die Probe eintreten. Anschließend an die Durchführung der Reaktion kann die Probe entnommen werden, ohne daß sie durch Moleküle der inkorporierten Substanz bzw. der an diese gebundenen Liganden verunreinigt ist. Anschließende Trennvorgänge entfallen. Selbstverständlich wird auch der Zugabevorgang für den Reaktionspartner eingespart, da der mit der Probe reagierende Partner bereits an der Gefäßwand gebunden vorliegt. Auf diese Weise werden biochemische Analysen erheblich vereinfacht und beschleunigt. Von großem Vorteil ist auch die sich erfindungsgemäß ergebende Mehrfachverwendbarkeit bei solchen freien Atomgruppen bzw. an diese gebundenen Liganden, die an der Reaktion nur katalytisch, also ohne Verbrauch teilnehmen. Insbesondere bei der medizinischen Labordiagnostik bei Massenreihenuntersuchungen, beispielsweise von Blutproben, können nacheinander in einem erfindungsgemäßen Reaktionsgefäß mehrere Proben zur Reaktion gebracht werden, wobei zwischen den Proben lediglich gespült werden muß. Bei klinischen Massenanalysen, z.B. in Blutbanken, lassen sich auf diese Weise erhebliche Kosteneinsparungen vornehmen, was insbesondere unter den heutigen Kostendämpfungsbestrebungen im Medizinwesen von erheblicher Bedeutung ist.

Vorteilhaft sind die Merkmale des Anspruches 2 vorgesehen. Zur Untersuchung von Patientenblut auf Krankheitsanzeichen wird Blutserum benötigt, welches aus Vollblut unter Durchführung der Blutgerinnungsreaktion gewonnen wird. Diese kann durch chemische Substanzen erzwungen werden, was aber für die nachfolgenden Analysen des Serums von Nachteil ist. Daher wird der Ablauf der natürlichen Blutgerinnungsreaktion bevorzugt, welche durch mechanische Läsion der Thrombozyten mit nachfolgender Freigabe von Prothrombin als Starterenzym der Gerinnungskette in Gang gesetzt wird. Ein Reaktionsgefäß der eingangs genannten Art, das nach diesem Prinzip arbeitet, ist aus der DE-OS 25 45 749 bekannt. Bei diesem bekannten Reaktionsgefäß werden zur Läsion der Thrombozyten scharfkantige Partikel zugegeben. Diese müssen allerdings anschließend entfernt werden. Es ist also ein Zugabe- und ein Trennvorgang erforderlich. Erfindungsgemäß wird die Läsion der Thrombozyten durch hydrophil ausgebildete reaktive Atomgruppen an der Gefäßwand bewirkt, die den Kontakt der Thrombozyten mit der Gefäßwand ermöglichen, welcher ansonsten an dem hydrophoben thermoplastischen Kunststoff nur äußerst langsam erfolgt. Dadurch wird auf einfache Weise ohne irgendwelche Zugabe in das Reaktionsgefäß die Blutgerinnungsreaktion gestartet, die nun sehr schnell abläuft.

Vorteilhaft sind dabei die Merkmale des Anspruches 3 vorgesehen. Glykole weisen als reaktive Atomgruppen im Sinne der Erfindung verwendbare OH-Gruppen auf, die hydrophil wirken, also für die Blutgerinnungsreaktion einsetzbar sind und andererseits zum Anbinden gewünschter Reaktionspartner, beispielsweise zum Anbinden von Enzymen oder Eiweißkörpern geeignet sind. Die Einbindung von Glykolen in Kunststoffe der eingangs genannten Art macht keinerlei Probleme. In Versuchen haben sich insbesondere Ethandiol und Propantriol als vorteilhaft erwiesen.

Weiterhin vorteilhaft sind die Merkmale des Anspruches 4 vorgesehen. Glyceringlykol wird nach entsprechenden Versuchen bevorzugt aufgrund seiner starken hydrophilen Wirkung und fehlender Lässigkeit.

Weiterhin vorteilhaft sind die Merkmale des Anspruches 5 vorgesehen. Der angegebene Gewichtsanteil Glyceringlykol, vorteilhaft 0,9 %, hat sich in Versuchen als Optimum herausgestellt. Abweichungen im Gewichtsanteil nach oben und unten im Bereich etwa einer Größenordnung führen nur zu unwesentlichen Verschlechterungen des Ergebnisses.

Weiterhin vorteilhaft sind bei einem Reaktionsgefäß zur Durchführung der Blutgerinnungsreaktion die Merkmale des Anspruches 6 vorgesehen. Auf diese Weise werden in die Oberfläche des Reaktionsgefäßes scharfkantige Körper eingebaut, die nach Art eines Schmirgeleffektes die lädierende Wirkung erhöhen. Auf diese Weise kann die Gerinnungszeit noch einmal um den Faktor 10 verkürzt werden.

Dabei sind vorteilhaft die Merkmale des Anspruches 7 vorgesehen. Silikatmehl kann sehr fein mit Teilchengrößen im Angströmbereich, also mit sehr großen Ober flächen, vorgesehen werden. Dieses sehr feine, bei Zugabe in die Flüssigkeit störende Material ist aber in die Kunststoffoberfläche eingebunden und gelangt nicht frei in das Serum.

Dabei sind vorteilhaft die Merkmale des Anspruches 8 vorgesehen. Dieser Gewichtsanteil hat sich als ausreichend herausgestellt und liegt derart niedrig, daß die gewünschten physikalischen Eigenschaften des Kunststoffes nicht verändert werden. Es lassen sich Zeiten für den Ablauf der Blutgerinnungsreaktion im Bereich 1 Minute erzielen.

Schließlich sind vorteilhaft die Merkmale des Anspruches 9 vorgesehen. Wie bereits eingangs erwähnt, können die in der Kunststoffoberfläche freiliegenden reaktiven Atomgruppen unmittelbar für eine biochemische Reaktion eingesetzt werden, beispielsweise in der zuvor geschildertn Weise als hydrophile Gruppen zum Start der Blutgerinnungsreaktion. Als wesentlicher Vorteil der Erfindung ergibt sich aber die Möglichkeit, an die reaktiven Atomgruppen biospezifisch aktive Liganden direkt oder über Spacer anzubinden, was aufgrund der hohen Verarbeitungstemperaturen bei der thermoplastischen Ausformung des Kunststoffes anschließend erfolgen muß. Derart präparierte Reaktionsgefäße sind in nahezu unübersehbarer Weise anwendbar. Aus der großen Zahl von Möglichkeiten seien nur einige Beispiele genannt:

An die reaktiven Atomgruppen können als Liganden angebunden werden beispielsweie Enzyme, Antkörper, Antigene, Hormone oder sonstige biospezifische Moleküle. Es können aber auch größere Fragmente biospezifisch aktiver Substanzen an die reaktiven Atomgruppen angebunden werden, beispielsweise Zellfragmente oder auch ganze lebende Zellen. Auf diese Weise können unter Beibehaltung der Vorteile der Erfindung beispielsweise Antigen-Antikörperreaktionen in völlig neuartiger Weise durchgeführt werden.

Im folgenden wird zunächst ein bevorzugtes Ausführungsbeispiel eines zum Ablauf der Blutoerinnungsreaktion vorgesehenen Reaktionsgefäßes beschrieben.

Das Reaktionsgefäß kann aus einem geeigneten thermoplastischen Kunststoff bestehen. Hierfür kommen in erster Linie Polystyrol, Polypropylen, Polyaethylen, Polycarbonat oder Polyacryl in Frage. Insbesondere haben sich Polystyrol oder Polypropylen als besonders geeignet erwiesen, die je nach Herstellungsverfahren oder nach den gewünschten mechanisch physikalischen Anforderungen an das Reaktionsgefäß wahlweise bevorzugt verwendbar sind. Im vorteilhaften Ausführungsbeispiel werden die beiden letztgenannten Kunststoffe wahlweise verwendet.

Das Reaktionsgefäß ist in der Regel zylindrisch ausgebildet und kann vorteilhaft bereits bei der Blutentnahme am Patienten als Spritzenzylinder verwendet werden. In ihm läuft die Blutgerinnungsreaktion ab. Vorteilhaft ist das Gefäß geometrisch derart ausgebildet, daß es anschließend zum Zentrifugieren und gegebenenfalls danach als Probenprimärgefäß im Analyseautomaten verwendbar ist, so daß Umfüllen und dergleichen Maßnahmen nicht erforderlich sind.

In einem bevorzugten Beispiel wird das Reaktionsgefäß wie folgt hergestellt:

Zu 25 kg handelsüblichem Polystyrol- oder Polypropylen-Granulat werden 225 g Glyceringlykol und 25 g Silikatmehl gegeben. Das Gemisch wird möglichst homogen vermischt und anschließend zum Plastifizieren erwärmt und in entsprechende Formen extrudiert. Nach Abkühlen und Entformung sind die Reaktionsgefäße fertig. Es stellt sich heraus, daß die Glykolmoleküle in den Kunststoff nun derart fest inkorporiert sind, daß keine Lässigkeit besteht. An den Kunststoffoberflächen liegen die Glykolmoleküle derart eingebettet, daß bei fester, nicht lässiger Inkorporierung des Molekülrestes OH-Gruppen an der Oberfläche freiliegen und dort als reaktive Atomgruppen im Sinne der Erfindung zur Verfügung stehen.

Die Blutserumgewinnung geschieht vorteilhaft wie folgt:

In das wie beschrieben hergestellten Reaktionsgefäß wird Vollblut eingegeben, vorteilhaft unmittelbar bei der Blutentnahme, wenn das Reaktionsgefäß als Spritzenzylinder ausgebildet ist. Die im Blut befindlichen Thrombocyten kommen aufgrund der hydrophilen Wirkung der freiliegenden OH-Gruppen der in den Kunststoff eingebundenen Glykole in innigen mechanischen Kontakt mit der Gefäßoberfläche, insbesondere mit den eingebetteten Silikatmehlspitzen. Dabei werden die Thrombocyten lädiert und setzen das Starterenzym Prothrombin frei. Nun läuft die Gerinnungskette ab.

Bei dem beschriebenen Reaktionsgefäß muß nach erstem Kontakt des Blutes mit dem Gefäß etwa 1 Minute abgewartet werden. Unmittelbar nach bzw. schon während der Blutentnahme ist die Gerinnungsreaktion also abgelaufen. Es kann sofort zentrifugiert werden, wobei sich die Gerinnungsfeststoffe und die Zellen absetzen. Anschließend wird das Serum dekantiert.

In dem angegebenen Herstellungsbeispiel für das Reaktionsgefäß ist die Beigabe von Glyceringlykol und Silikatmehl zum Kunststoffgranulat vor der Plastifizierung beschrieben. Alternativ läßt sich dieses Material auch vorgemischt als Granulat einsetzen und kann beispielsweise in dieser Form direkt vom Kunststoffhersteller produziert werden.

Das beschriebene Reaktionsgefäß zeichnet sich insbesondere auch durch gute Entsorgbarkeit aus. Die guten Entsorgungseigenschaften der verwendeten thermoplastischen Kunststoffe werden durch die erfindungsgemäßen Substanzbeigaben nicht gestört. Es läßt sich also beispielsweise eine schadstofffreie Verbrennung durchführen.

Reaktionsgefäße für weitere Anwendungen im Rahmen der vorliegenden Erfindung werden auf entsprechende Weise hergestellt. In besonders vorteilhafter Weise sind für komplexere chemische Reaktionen Eiweißkörper als auf die Probe wirkende Reaktionspartner an die Gefäßwand gebunden, und zwar an die freiliegenden reaktiven Atomgruppen der inkorporierten Substanz. Beispielsweie können für Antigen-Antikörperreaktionen Zellfragmente an die reaktiven Atomgruppen gebunden werden. Für Enzymreaktionen können Enzyme gebunden werden. Sogar lebende Zellen sind auf diese Weise an die Kunststoffoberfläche bindbar und stehen mit dem vollen Umfang ihrer biochemischen Aktivitäten zur Verfügung.

Die Anbindung der biospezifisch wirkenden Liganden an die in der Kunststoffwand freiliegenden aktiven Atomgruppen der dem Kunststoff beigegebenen Substanz kann unmittelbar erfolgen oder in bekannter Weise über Spacer, falls dies für die durchzuführende Reaktion erforderlich ist.

Als dem Kunststoff zugegebene Substanz mit freibleibenden reaktiven Atomgruppen können für diese Zwecke insbesondere die eingangs erwähnten Glykole verwendet werden, deren freie OH-Gruppen für vielfältige Anbindungszwecke bestens geeignet sind.

Im folgenden wird als Beispiel die Anbindung von Enzymen beschrieben:

Dabei werden zunächst die freien OH-Gruppen von an den Kunststoff gebundenen Glykolen durch Cyanbromid (BrCN) aktiviert. Die OH-Gruppen reagieren mit Cyanbromid unter Ausbildung reaktiver Imidocarbonreste. Diese elektrophilen Gruppen verbinden sich kovalent mit nucleophilen Gruppen des zu immobilisierenden Enzyms.

Das zu bindende Enzym wird in gepufferter Lösung, deren pH auf das pH-Optimum des Enzyms eingestellt ist, eingebracht. Die Fixierung erfolgt ohne weiteres Zutun während einer Inkubationszeit, deren optimale Temperatur und Dauer für unterschiedliche Enzyme verschieden sein kann. Nicht fixiertes überschüssiges Enzym wird mit der Pufferlösung ausgewaschen.

Die Menge des gebundenen Enzyms wird durch Differenzmessung der Enzymaktivität in der Enzymlösung vor und nach dem Bindungsvorgang ermittelt, wobei die Restaktivitäten im Waschpuffer mit zu berücksichtigen sind.

Die Konservierung der auf diese Weise am Kunststoff fixierten Enzyme kann vorteilhaft durch Lyophilisierung (Gefriertrocknung) erfolgen. Eine solche Konservierung ist erforderlich, wenn das nun fertiggestellte reaktionsbereite Reaktionsgefäß nicht sofort zur Reaktion verwendet wird. Zur Aufbewahrung des fertiggestellten Reaktionsgefäßes mit lyophilisierten Enzymen bis zu seiner Anwendung ist es vorteilhaft, die reaktive Gefäßfläche unter Vakuum zu halten. Übliche Techniken der Lagerung unter Vakuum können hier Verwendung finden.

Antikörper, Hormone oder beispielsweise die eingangs erwähnten lebenden Zellen können in weitgehend ähnlicher Weise gebunden werden. Zum späteren Einsatz bei der Radioimmunassay- oder der Enzymimmunassaytechnik können die zu fixierenden Liganden zuvor mit Radioisotopen oder Enzymen markiert werden.

Die Anwendung der beschriebenen Ausführungsformen des erfindungsgemäßen Reaktionsgefäßes ist insbesondere für die medizinische Laboratoriumsdiagnostik vorgesehen (beispielsweise im Falle des dargestellten Ausführungsbeispieles der Anwendung für die Blutgerinnungsreaktion). Weitere Anwendungen liegen auf dem Feld der biologischen Labordiagnostik für die unterschiedlichsten Zwecke. Es sind mit großem Vorteil aber auch Anwendungen bei biotechnologischen Prozessen möglich. Solche Anwendungen bestehen beispielsweise bei der Arzneimittel- und Nahrungsmittelherstellung sowie bei biologischen Entsorgungsverfahren. Insbesondere bei solchen Verfahren sind lebende Zellen als Liganden mit Vorteil einsetzbar.

## Patentansprüche

1. Reaktionsgefäß aus chemisch inertem thermoplastischem Kunststoff zur Durchführung chemischer Reaktionen mit biospezifischen Verbindungen vorzugsweise für die medizinische Laboratoriumsdiagnostik, dadurch gekennzeichnet, daß der Kunststoff durch vor seiner thermoplastischen Ausformung erfolgte Beigabe einer nicht lässig inkorporierbaren Substanz derart ausgebildet ist, daß nach dem Ausformen des Kunststoffes wenigstens an der Gefäßinnenfläche inkorporierte Substanzmoleküle mit reaktiven Atomgruppen freiliegen.

2. Reaktionsgefäß nach Anspruch 1, dadurch gekennzeichnet, daß die reaktiven Gruppen hydrophil ausgebildet sind.

3. Reaktionsgefäß nach einem der vorhergehenden Ansprüche, dadurch gekennzeichnet, daß die Beigabesubstanz ein Glykol ist.

4. Reaktionsgefäß nach Anspruch 2, dadurch gekennzeichnet, daß die Beigabesubstanz Glyceringlykol ist.

5. Reaktionsgefäß nach Anspruch 4, dadurch gekennzeichnet, daß der Gewichtsanteil des Glyceringlykols etwas unter 1 % liegt.

6. Reaktionsgefäß nach Anspruch 2, dadurch gekennzeichnet, daß dem Kunststoff vor der thermoplastischen Ausformung ein geringer Anteil an chemisch inertem scharfkantigem Hartstoffpulver zugegeben ist.

7. Reaktionsgefäß nach Anspruch 6, dadurch gekennzeichnet, daß Silikatmehl zugegeben ist.

8. Reaktionsgefäß nach Anspruch 7, dadurch gekennzeichnet, daß der Gewichtsanteil an Silikatmehl ca. 1 %o beträgt.

9. Reaktionsgefäß nach einem der Ansprüche 1 bis 5, dadurch gekennzeichnet, daß die reaktiven Atomgruppen nach der Ausformung des Reaktionsgefäßes kovalent angebundene biospezifisch aktive Liganden tragen.

## Claims

1. Reaction vessel of chemically inert thermoplastic plastics material for conducting chemical reactions with biospecific compounds, preferably for medical laboratory diagnostic purposes, characterised in that the plastics material is so constructed due to the addition, prior to its thermoplastic shaping, of a substance, which can be incorporated such that it is not released, that after the shaping of the plastics material reactive atom groups of incorporated molecules of the substance are exposed, at least at the inner surface of the vessel.

2. Reaction vessel as claimed in claim 1, characterised in that the reactive groups are hydrophilic.

3. Reaction vessel as claimed in one of the preceding claims, characterised in that the added substance is a glycol.

4. Reaction vessel as claimed in claim 2, characterised in that the added substance is glycerine glycol.

5. Reaction vessel as claimed in claim 4, characterised in that the proportion by weight of the glycerine glycol is somewhat below 1%.

6. Reaction vessel as claimed in claim 2, characterised in that before the thermoplastic shaping a small proportion of chemically inert, sharp-edged hard material powder is added to the plastics material.

7. Reaction vessel as claimed in claim 6, characterised in that silicate flour is added.

8. Reaction vessel as claimed in claim 7, characterised in that the proportion by weight of silicate flour is about 1%o.

9. Reaction vessel as claimed in one of claims 1 to 5, characterised in that the reactive atom groups carry covalently bonded, biospecifically active ligands after the shaping of the reaction vessel.


**Revendications**

1. Récipient de réaction en matière thermoplastique chimiquement inerte pour la conduite de réactions chimiques avec des composés biospécifiques, de préférence pour le diagnostic médical au laboratoire, caractérisé en ce que, par une addition de substance incorporable sans migration possible, effectuée avant son formage thermoplastique, la matière plastique est réalisée de façon telle qu'après son démoulage, des molécules de la substance incorporée portant des groupes d'atomes réactifs soient libres au moins à la surface interne du récipient.

2. Récipient de réaction suivant la revendication 1, caractérisé en ce que les groupes réactifs ont une constitution hydrophile.

3. Récipient de réaction suivant l'une des revendications précédentes, caractérisé en ce que la substance ajoutée est un glycol.

4. Récipient de réaction suivant la revendication 2, caractérisé en ce que la substance ajoutée est le glycéroglycol.

5. Récipient de réaction suivant la revendication 4, caractérisé en ce que la proportion en poids de glycéroglycol est un peu inférieure à 1 %.

6. Récipient de réaction suivant la revendication 2, caractérisé en ce qu'on ajoute à la matière plastique avant le formage thermoplastique de faibles proportions d'une substance dure en poudre à arêtes vives chimiquement inerte.

7. Récipient de réaction suivant la revendication 6, caractérisé en ce qu'on ajoute un silicate en poudre.

8. Récipient de réaction suivant la revendication 7, caractérisé en ce que la proportion en poids du silicate en poudre s'élève à environ 1 pour 1000.

9. Récipient de réaction suivant l'une des revendications 1 à 5, caractérisé en ce que les groupes d'atomes réactifs portent, après la mise en forme du récipient de réaction, des ligands doués d'activité biospécifique liés par covalence.